(19) Europäisches Patentamt
European Patent Office
Office européen des brevets

(11) Veröffentlichungsnummer: **0 011 606**
**A2**

# (12) EUROPÄISCHE PATENTANMELDUNG

(21) Anmeldenummer: **79810153.1**

(22) Anmeldetag: **15.11.79**

(51) Int. Cl.³: **A 61 K 31/445**
//(A61K31/445, 31/445)

(30) Priorität: **21.11.78 CH 11918/78**

(43) Veröffentlichungstag der Anmeldung:
**28.05.80 Patentblatt 80/11**

(84) Benannte Vertragsstaaten:
**BE CH DE FR GB IT LU NL SE**

(71) Anmelder: **CIBA-GEIGY AG**
**Patentabteilung Postfach**
**CH-4002 Basel(CH)**

(72) Erfinder: **Waldmeier, Peter, Dr.**
**Passwangstrasse 17**
**CH-4153 Reinach(CH)**

(72) Erfinder: **Delini-Stula, Aleksandra, Dr.**
**Neubadstrasse 72**
**CH-4054 Basel(CH)**

## (54) Pharmazeutische Präparate mit zentraldämpfender und antipsychotischer Wirksamkeit.

(57) Die Erfindung betrifft pharmazeutische Präparate mit zentraldämpfender und antipsychotischer Wirksamkeit, die als Wirkstoffe ein zentraldämpfend und antipsychotisch wirksames, in 4-Stellung basisch substituiertes Butyrophenenderivat, insbesondere ein solches der algemeinen Formel I

(I)

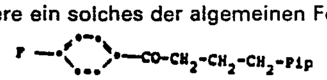

in welcher Pip einen 4,4-disubstituierten oder 4-substituierten Piperidinorest bedeutet, oder ein pharmazeutisch annehmbares Säureadditionssalz eines solchen und ein gegebenenfalls substituiertes C-(2-Benzofuranyl)- piperidin oder C-(2-Benzofuranyl)-tetrahydropyridin der allgemeinen Formel II

(II)

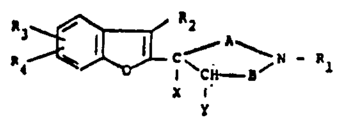

in welcher $R_1$ Wasserstoff, eine Alkylgruppe mit höchstens 4 Kohlenstoffatomen, die Allyl-, 3-Oxobutyl-, 3-Hydroxybutyl-, 2-Propinyl- oder Cyclopropylmethylgruppe, $R_2$ Wasserstoff oder eine Methylgruppe, $R_3$ Wasserstoff, Halogen bis Atomnummer 35, eine niedere Alkyl- oder Alkoxygruppe, die Trifluormethylgruppe oder eine Cycloalkylgruppe mit 5-8 Kohlenstoffatomen, $R_4$ Wasserstoff eine niedere Alkylgruppe oder Halogen bis Atomnummer 35, oder als $R_3$ und $R_4$ zusammen einen 1,3-Butadienylenrest in 4,5-Stellung, entsprechend einem ankondensierten Benzolring, oder einen Trimethylenrest in 5,6-Stellung, A die Aethylengruppe und B die Methylengruppe oder A die Methylengruppe und B die Aethylengruppe, und X und Y je Wasserstoff oder zusammen eine zusätzliche Bindung bedeuten, oder ein pharmazeutisch annehmbares Säureadditionssalz eines solchen enthalten.

EP 0 011 606 A2

4-12124 /=

## Pharmazeutische Präparate mit zentraldämpfender und antipsychotischer Wirksamkeit

Die vorliegende Erfindung betrifft neue pharmazeutische Präparate, die als pharmakologische Wirkstoffe ein zentraldämpfend-antipsychotisch wirksames, basisch substituiertes Butyrophenon und ein gegebenenfalls in bestimmter Weise substituiertes C-(2-Benzofuranyl)-piperidin oder -tetrahydropyridin oder pharmazeutisch annehmbare Säureadditionssalze dieser Basen enthalten.

Mit den neuen pharmazeutischen Präparaten können im wesentlichen die gleichen Krankheitszustände behandelt werden wie mit den bekannten, basisch substituierten Butyrophenonen allein, wobei jedoch die Dosierung dieser Wirstoffe herabgesetzt werden kann.

Es ist bekannt, dass basisch substituierte Butyrophenone, insbesondere substituierte p-Fluor-4-piperidinobutyrophenone wie 4-[4-(p-Chlorphenyl)-4-hydroxy-piperidino]-p-fluorbutyrophenon, F.N. Haloperidol, p-Fluor-4-[4-hydroxy-4-(m-trifluormethylphenyl)-piperidino]-butyrophenon, F.N. Trifluperidol, und p-Fluor-4-[4-(2-oxo-2,3-dihydro-1H-benzimidazol-1-yl)-piperidino]-butyrophenon, F.N. Benperidol, sehr starke zentraldämpfende und antipsychotische Wirksamkeit besitzen und beispielsweise zur Behandlung von psychomotorischen Erregungszuständen, z.B. bei zerebraler Arteriosklerose und seniler Demenz, von Schizophrenien, manischen Psychosen und paranoidhalluzinatorischen Syndromen in der Psychiatrie breite Anwendung finden.

Weiter ist z.B. aus der deutschen Offenlegungsschrift 2 408 476 und aus der englischen Patentschrift 1 465 581 bekannt, dass C-(2-Benzofuranyl)-piperidine und -tetrahydropyridine, die in 1-Stellung und/oder im aromatischen Ring in bestimmter Weise substituiert sein können, an der Ratte und an weiteren Versuchstierarten bei oraler oder subcutaner Applikation die Aufnahme von Serotonin in die Mittelhirnsynaptosomen hemmen und ebenfalls an Ratten bei gleicher Applikationsweise auch selektiv die A-Form der Monoaminoxidase sowie die Aufnahme von Noradrenalin ins Herz hemmen. Die genannten C-(2-Benzofuranyl)-piperidine und -tetrahydropyridine antagonisieren ferner bei intraperitonealer Verabreichung an der Ratte die Wirkung von Tetrabenazin und besitzen somit ein Wirkungsspektrum, das auf ihre Verwendbarkeit als Antidepressiva schliessen lässt.

Es wurde nun überraschenderweise gefunden, dass für die zentraldämpfenden und antipsychotischen Butyrophenonderivate charakteristische pharmakologische Wirkungen, wie die kataleptische Wirkung und besonders ausgeprägt die Antagonisierung des durch die Verabreichung von Apomorphin ausgelösten stereotypen Verhaltens der Ratten, durch gegebenenfalls substituierte C-(2-Benzofuranyl)-piperidine, welche für sich allein in keiner Dosierung entsprechende Wirkungen zeigen, potenziert werden. Beispielsweise ergeben 0,3 mg/kg p.o. Haloperidol zusammen mit 10,0 mg/kg p.o. 4-(5,6-Dimethyl-2-benzofuranyl)-piperidin-hydrochlorid (DMBP-hydrochlorid) an der Ratte eine etwas rascher einsetzende und früher abklingende, aber im Ganzen ungefähr gleich starke kataleptische Wirkung wie 0,5 mg/kg p.o. Haloperidol allein. Die durch Apomorphin

ausgelöste Stereotypie bei der Ratte wurde durch 0,3 mg/kg p.o. Haloperidol allein schwächer und durch 0,45 mg/kg p.o. Haloperidol allein stärker antagonisiert als durch 0,06 mg/kg p.o. Haloperidol nach Behandlung mit 10,0 mg/kg p.o. DMPB-hydrochlorid. Die Potenzierung von Haloperidol zeigt sich in diesem Test somit in einer ca. 6-fachen Verminderung der wirkungsgleichen Dosis, während die Potenzierung von Haloperidol im Katalepsie-Test weniger stark ist und bloss einer ca. 1,7-fachen Verminderung der wirkungsgleichen Dosis entspricht.

DMBP-hydrochlorid potenziert auch die durch verschiedene Dosen von Haloperidol bewirkte Steigerung der Konzentration der Homovanillinsäure (HVA) im Striatum, welche als Index für den Aktivierungsgrad von dopaminergen Neuronen betrachtet werden kann. So bewirkt in Ratten, die mit der in diesem Test völlig unwirksamen Dosis von 10 mg/kg per os DMBP-hydrochlorid behandelt waren, 0,03 mg/kg p.o. Haloperidol bereits eine gleich starke Steigerung der 2 Stunden später nach Tötung der Tiere bestimmten Konzentration von HVA wie 0,1 mg/kg p.o. Haloperidol an nicht-vorbehandelten Ratten, und 0,1 mg/kg p.o. an vorbehandelten Ratten eine annähernd gleich starke Steigerung wie 0,3 mg/kg p.o. Haloperidol an nicht-vorbehandelten Ratten, entsprechend einer Senkung der wirkungsgleichen Haloperidol-Dosen durch Vorbehandlung mit DMBP-hydrochlorid auf ca. ein Drittel. Bei gleichbleibender Dosierung des Haloperidol von 0,1 mg p.o. bewirken verschiedene, 30 Minuten vorher p.o. verabreichte Dosen von DMBP-hydrochlorid die in der nachstehenden Tabelle 1 angegebenen Steigerungen der Konzentration von HVA und 3,4-Dihydroxyphenylessigsäure (DOPAC) im Striatum der 2 Stunden nach der Haloperidol-Verabreichung getöteten Ratten (n = 4-5 pro Dosis)

Tabelle 1

| Testtyp | H mg/kg p.o. | D mg/kg p.o. | HVA ng/g | % von Kon | DOPAC ng/g | % von Kon |
|---|---|---|---|---|---|---|
| Kon | - | - | 332 ± 19 | 100 | 1155 ± 47 | 100 |
| H | 0.1 | - | 462 ± 38 | 139 | 1481 ± 64 | 128 |
| D | - | 1 | 309 ± 15 | 93 | 1006 ± 39 | 87 |
| H + D | 0.1 | + 1 | 834 + 84 | 251 | 2108 ± 92 | 183 |
| D | - | 3 | 344 ± 12 | 104 | 994 ± 72 | 86 |
| H + D | 0.1 | + 3 | 1202 ± 117 | 362 | 2495 ± 159 | 216 |
| D | - | 10 | 318 ± 19 | 96 | 974 ± 56 | 84 |
| H + D | 0.1 | + 10 | 1198 ± 82 | 361 | 2697 ± 193 | 234 |

Kon = unbehandelte Kontrolltiere, H = Haloperidol,
D = DMBP-Hydrochlorid

Durch Versuche mit $H^3$-markiertem Haloperidol konnte weiterhin festgestellt werden, dass DMBP keinen Einfluss auf den Metabolismus von Haloperidol im Striatum ausübt und die Potenzierung deshalb nicht auf der Beeinflussung von Konzentration und Abbau von Haloperidol durch DMBP beruhen dürfte.

Aufgrund dieser experimentellen Befunde kann angenommen werden, dass bei gleichbleibender oder sogar verstärkter antipsychotischer Wirksamkeit die klinischen Dosen von Haloperidol und ähnlichen Neuroleptica herabgesetzt werden können, und als Folge eine Verminderung der Nebenwirkungen eintritt.

Gegenstand der vorliegenden Erfindung sind nun pharmazeutische Präparate, die als Wirkstoff ein zentraldämpfend und antipsychotisch wirksames, in 4-Stellung basisch substituiertes Butyrophenonderivat I oder ein pharmazeutisch annehmbares Säureadditionssalz eines solchen und ein gegebenenfalls substituiertes C-(2-Benzofuranyl)-piperidin oder C-(2-Benzofuranyl)-tetrahydropyridin der allgemeinen Formel II

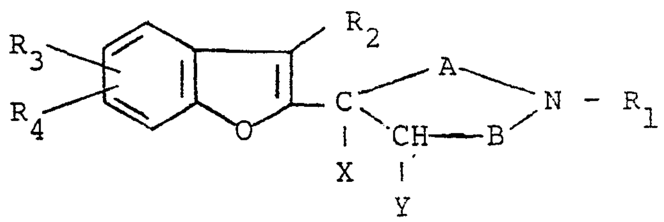

(II)

in welcher $R_1$ Wasserstoff, eine Alkylgruppe mit höchstens 4 Kohlenstoffatomen, die Allyl-, 3-Oxobutyl-, 3-Hydroxybutyl-, 2-Propinyl-oder Cyclopropylmethylgruppe, $R_2$ Wasserstoff oder eine Methylgruppe, $R_3$ Wasserstoff, Halogen bis Atomnummer 35, eine niedere Alkyl- oder Alkoxygruppe, die Trifluormethylgruppe oder eine Cycloalkylgruppe mit 5-8 Kohlenstoffatomen, $R_4$ Wasserstoff, eine niedere Alkylgruppe oder Halogen bis Atomnummer 35, oder als $R_3$ und $R_4$ zusammen einen 1,3-Butadienylenrest in 4,5-Stellung, entsprechend einem ankondensierten Benzolring, oder einen Trimethylenrest in 5,6-Stellung, A die Aethylengruppe und B die Methylengruppe oder A die Methylengruppe und B die Aethylengruppe, und X und Y je Wasserstoff oder zusammen eine zusätzliche Bindung bedeuten, oder ein pharmazeutisch annehmbares Säureadditionssalz eines solchen enthalten.

Als Verbindungen der allgemeinen Formel II kommen insbesondere solche in Betracht, in denen $R_1$ Wasserstoff, ein Alkylrest mit höchstens 4 Kohlenstoffatomen, ein 2-Propinyl- oder Cyclopropylmethylrest, $R_2$ Wasserstoff, $R_3$ Wasserstoff, Halogen bis Atomnummer 35, eine Methyl-, Methoxy- oder Cyclohexylgruppe, vorzugsweise in 5-Stellung, $R_4$ Wasserstoff, eine Methylgruppe, Chlor oder Brom, A

eine Aethylengruppe und B eine Methylengruppe ist und X und Y zusammen eine zusätzliche Bindung oder vorzugsweise je ein Wasserstoffatom bedeuten. Besonders eignen sich solche Verbindungen der allgemeinen Formel II und des vorstehend hervorgehobenen Typus, in denen die Wirkungsqualität der Serotonin-Aufnahmehemmung im Vergleich zu den andern Wirkungsqualitäten besonders hervortritt. Beispielsweise kommen als Verbindungen der allgemeinen Formel II das 4-(6-Chlor-2-benzofuranyl)-piperidin und in erster Linie das 4-(5,6-Dimethyl-2-benzofuranyl)-piperidin und deren pharmazeutisch annehmbare Säureadditionssalze in Betracht.

Die in 4-Stellung basisch substituierten Butyrophenon-derivate sind insbesondere solche der allgemeinen Formel I

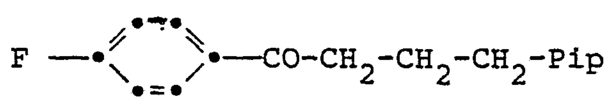 (I)

in welcher Pip einen 4,4-disubstituierten oder 4-substituierten Piperidinorest, insbesondere einen 4-Hydroxy-4-arylpiperidinorest, vor allem einen in 4-Stellung durch Hydroxy und Halogenphenyl, Trifluormethylphenyl oder Niederalkylphenyl substituierten Piperidinorest, wie den 4-Hydroxy-4-(p-chlorphenyl)-piperidino-, 4-Hydroxy-4-[m-(trifluormethyl)-phenyl]-piperidino- oder 4-Hydroxy-4-(p-tolyl)-piperidinorest, weiter den 4-Carbamoyl-4-piperidino-piperidinorest bzw. den 4-(2-Oxo-2,3-dihydro-1H-benzimidazol-1-yl)-piperidinorest bedeutet.

In den erfindungsgemässen pharmazeutischen Präparaten kann das Mengenverhältnis des zentraldämpfenden und antipsychotischen Butyrophenon-derivat I zur Verbindung der allgemeinen Formel II und/oder von pharmazeutisch annehmbaren Säureadditionssalzen dieser Wirkstoffe innerhalb weiter Grenzen variieren. Es liegt im allgemeinen zwischen 1 zu 2 und 1 zu 100, vorzugsweise zwischen 1 zu 4 und 1 zu 50, und für die beiden nachstehend genannten Wirkstoffe insbesondere zwischen 1 zu 10 und 1 zu 50.

Die absolute Dosierung der beiden Wirkstofftypen in den erfindungsgemässen Präparaten variert ebenfalls stark und hängt insbesondere von der relativen Potenz der verwendeten Wirkstoffe, in erster Linie von derjenigen der verwendeten Butyrophenon-derivate ab. Doseneinheiten oder passende Mengen von nicht-einzeldosierten Arzneiformen, wie z.B. 1 ml Tropfen oder 5 ml Sirup, zur Verabreichung an grössere Warmblüter von durchschnittlich ca. 70 kg Körpergewicht enthalten beispielsweise 0,25 bis 2,5 mg Haloperidol oder eine wirkungsgleiche, meist höhere, Menge eines andern zentraldämpfenden und/oder antipsychotisch wirksamen Butyrophenon-derivates I oder eines pharmazeutisch annehmbaren Säureadditionssalzes eines solchen und 5 bis 50 mg, vorzugsweise 10 bis 25 mg einer Verbindung der allgemeinen Formel II oder eines pharmazeutisch annehmbaren Säureadditionssalzes einer solchen. So enthalten erfindungsgemässe pharmazeutische Präparate beispielsweise 0,25 bis 2,5 mg, vorzugsweise 0,5 bis 1 mg Haloperidol zusammen mit 5 bis 50 mg, vorzugsweise 10 bis 25 mg 4-(5,6-Dimethyl-2-benzofuranyl)-piperidin, gegebenenfalls in Form von pharmazeutisch annehmbaren Säureadditionssalzen. Vorzugsweise erhalten die erfindungsgemässen Präparate die Wirkstoffe zusammen mit üblichen Träger- und/oder Hilfsstoffen.

Von denerfindungsgemässen pharmazeutischen Präparaten werden im allgemeinen zweimal oder vorzugsweise dreimal täglich ein bis zwei Doseneinheiten oder entsprechende Mengen nicht-einzeldosierter Arzneiformen verabreicht, so dass die Tagesdosen vorzugsweise dem 2- bis 6-fachen der oben angegebenen Einzeldosen entsprechen. Als Erhaltungsdosen kommen aber auch gegenüber den obengenannten wesentlich reduzierte Dosen in Betracht.

Ebenfalls Gegenstand der vorliegenden Erfindung ist die vorgenannte Verwendung der pharmazeutischen Präparate zur Behandlung der weiter oben genannten Krankheitszustände. Ferner umfasst die Erfindung auch die separate, gleichzeitige oder zeitlich gestaffelte Verabreichung eines oder mehrerer Butyrophenon-derivate I und einer oder mehrerer Verbindungen der allgemeinen Formel II oder von pharmazeutisch annehmbaren Salzen dieser Wirkstoffe in Form von geeigneten Applikationsformen, insbesondere Doseneinheitsformen, in Mengen, die mit den in den vorgenannten Kombinationspräparaten enthaltenen Dosen übereinstimmen, zur Behandlung der weiter oben genannten Krankheitszustände.

Die erfindungsgemässen pharmazeutischen Präparate sindübliche Applikationsformen zur enteralen Verabreichung, wie z.B. Tabletten, Dragées, Kapseln, Suppositorien, Tropflösungen oder Sirupe oder parenteralen Verabreichung, insbesondere wässrige Injektionslösungen zur intramuskulären, intravenösen oder subcutanen Verabreichung. Feste Doseneinheitsformen können im wesentlichen homogene Mischungen der Wirkstoffe enthalten, oder z.B. im Kern den einen Wirkstoff, und im Mantel oder in einem aufgepressten, sogenannten Punkt den andern Wirkstoff enthalten, wobei auch in an sich bekannter Weise eine zeitliche Staffelung der

Wirkstoffaufnahme bewirkt werden kann, indem z.B. der Kern oder der sogenannte Punkt, in welchem sich vorzugsweise das viel niedriger dosierte Butyrophenon-derivat I befindet, mit einem magensaftresistenten Ueberzug versehen wird.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt. So kann man Tabletten bzw. Dragée-Kerne zur oralen Anwendung erhalten, indem man die Wirkstoffe mit festen Trägerstoffen kombiniert, ein erhaltenes Gemisch gegebenenfalls granuliert, und das Gemisch bzw. Granulat, wenn erwünscht oder notwendig nach Zugabe von geeigneten Hilfsstoffen, zu Tabletten oder Dragée-Kernen verarbeitet.

Als Trägerstoffe kommen beispielsweise in Betracht: Zucker, z.B. Lactose, Saccharose, Mannit oder Sorbit, übliche Cellulosepräparationen, Calciumphosphate, z.B. Tricalcium- oder Calciumhydrogenphosphat, Bindemittel, wie Stärkekleister, z.B. auf Basis von Mais-, Weizen-, Reis- oder Kartoffelstärke, Gelatine, Traganth, Methylcellulose, Natriumcarboxycellulose und/oder Polyvinylpyrrolidon, ferner Carboxymethylstärke, quervernetztes Polyvinylpyrrolidon, Agar, Alginsäure bzw. ein Alginat, z.B. Natriumalginat, und/oder, wenn erwünscht, Sprengmittel, wie die obgenannten Stärken. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabi-

BAD ORIGINAL

schen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Durch Einarbeiten eines oder mehrerer Wirkstoffe in ein geeignetes Trägermaterial, das eine langsame Abgabe des oder der Wirkstoffe bewirkt, kann die Wirkungsdauer einer oder mehrerer an sich kurz wirksamer Komponenten verlängert werden. Den Tabletten oder Dragée-Ueberzügen können Farbstoffe oder Pigmente, z.B. zur Identifizierung oder zur Kennzeichnung verschiedener Kombinationen von Wirkstoffdosen, beigefügt werden. Gewünschtenfalls kann man auch erhaltene Tabletten zur Herstellung von sogenannten Lacktabletten mit entsprechenden, gegebenenfalls Magensaft-resistenten Ueberzügen versehen.

Weitere oral anwendbare pharmazeutische Präparate sind Steckkapseln aus Gelatine, sowie weiche, geschlossene Kapseln aus Gelatine und einem Weichmacher, wie Glyerin oder Sorbitol. Die Steckkapseln können die Wirkstoffe in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln sind die Wirkstoffe vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Für nicht einzeldosierte Flüssigkeiten zur oralen Einnahme, wie Sirups und Elixiere, wird die Konzentration der Wirkstoffe derart gewählt, dass eine Einzeldosis leicht abgemessen werden kann, z.B. als Inhalt eines Mess-

löffels von z.B. 5ml, oder auch als Mehrfaches dieses Volumens. Als Sirups eignen sich beispielsweise Lösungen von wasserlöslichen oder Suspensionen von unlöslichen, aber resorbierbaren Säureadditionssalzen in wässrigen Lösungen von Zuckern und/oder Alkanpolyolen, wie Rohrzucker bzw. Sorbit oder Glycerin, Geschmacks- und Aromastoffen sowie gegebenenfalls Konservierungs- und Stabilisierungsmitteln. Elixiere sind wässrig-alkoholische Lösungen der erfindungsgemäss verwendbaren Wirkstoffe oder von pharmazeutisch annehmbaren Salzen derselben, die ebenfalls die bei den Sirups genannten Zusätze enthalten können. Als weitere orale Applikationsformen seien Tropflösungen genannt, die meist einen höheren Alkoholgehalt und zugleich einen höheren Wirkstoffgehalt aufweisen, so dass eine Einzeldosis z.B. als 10 bis 50 Tropfen abgemessen werden kann.

Als rektal anwendbare pharmazeutische Präparate kommen z.B. Suppositorien in Betracht, welche aus einer Kombination der Wirkstoffe mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die aus einer Kombination der Wirkstoffe mit einer Grundmasse bestehen; als Grundmassenstoffe kommen z.B. flüssige Triglyceride, Polyäthylenglykole oder Paraffinkohlenwasserstoffe in Frage.

Ampullenlösungen zur parenteralen, insbesondere intramuslulären oder intravenösen, ferner subcutanen Verabreichung enthalten die erfindungsgemäss verwendbaren Wirkstoffe in einer Gesamtkonzentration von beispielsweise 0,275 bis 5,25 % als wässrige, mit Hilfe von üblichen Lösungsvermittlern und/oder Emulgiermitteln, sowie gegebenenfalls von Stabilisierungsmitteln bereitete Dispersion,

oder vorzugsweise als wässrige Lösung von pharmazeutisch annehmbaren, wasserlöslichen Säureadditionssalzen.

Die nachfolgenden Beispiele sollen die Herstellung von zwei typischen Applikationsformen erläutern, jedoch keineswegs die einzigen Ausführungsformen von solchen darstellen.

Beispiel 1

Tabletten, enthaltend 0,025 g 4-(5,6-Dimethyl-2-benzofuranyl)-piperidin-hydrochlorid und 0,001 g 4-[4-(p-Chlorphenyl)-4-hydroxy-piperidino]-p-fluorbutyrophenon, können z.B. wie folgt hergestellt werden:

Zusammensetzung (für 1000 Tabletten)

| | |
|---|---|
| 4-(5,6-Dimethyl-2-benzofuranyl)-piperidin-hydrochlorid | 25 g |
| 4-[4-(p-Chlorphenyl)-4-hydroxy-piperidino]-p-fluorbutyrophenon | 1 g |
| Lactose | 50 g |
| Weizenstärke | 90 g |
| Kolloidale Kieselsäure | 20 g |
| Magnesiumstearat | 4 g |
| Talk | 10 g |
| Wasser | q.s. |

Die Wirkstoffe werden mit einem Teil der Weizenstärke, mit der Lactose und der kolloidalen Kieselsäure vermischt und das Gemisch durch ein Sieb getrieben. Ein weiterer Teil der Weizenstärke wird mit der fünffachen Menge Wasser auf dem Wasserbad verkleistert und die obige Pulvermischung mit diesem Kleister angeknetet, bis eine schwach plastische Masse entstanden ist. Die plastische Masse wird durch ein Sieb von etwa 3 mm Maschenweite gedrückt, getrocknet und das trockene Granulat nochmal durch ein Sieb getrieben. Darauf werden die restlichen Weizenstärke, der Talk und das Magnesiumstearat zugemischt und die erhaltene Mischung zu Tabletten von 0,2 g verpresst.

Beispiel 2

Dragées, enthaltend 0,025 g 4-(5,6-Dimethyl-2-benzofuranyl)-piperidin-hydrochlorid und 0,001 g 4-[4-(p-Chlorphenyl)-4-hydroxy-piperidino]-p-fluorbutyrophenon, können z.B. folgendermassen hergestellt werden:

1000 g der z.B. nach Beispiel 1 erhältlichen Tabletten werden in üblicher Weise in zwei Stufen mit einem zuckerhaltigen Sirup dragiert. Als solcher wird in der ersten Stufe ein Sirup bestehend aus einem Teil Zucker und zwei Teilen Wasser unter Zusatz von Talk (18%), Polyvinylpyrrolidon (1,5%) und Polyäthylenglykol 6000 (1%), und in der zweiten Stufe ein reiner Zuckersirup verwendet.

## Patentansprüche

1. Pharmazeutische Präparate mit zentraldämpfender und antipsychotischer Wirksamkeit, die als Wirkstoffe ein zentraldämpfend und antipsychotisch wirksames, in 4-Stellung basisch substituiertes Butyrophenonderivat I oder ein pharmazeutisch annehmbares Säureadditionssalz eines solchen und ein gegebenenfalls substituiertes C-(2-Benzofuranyl)-piperidin oder C-(2-Benzofuranyl)-tetrahydropyridin der allgemeinen Formel II

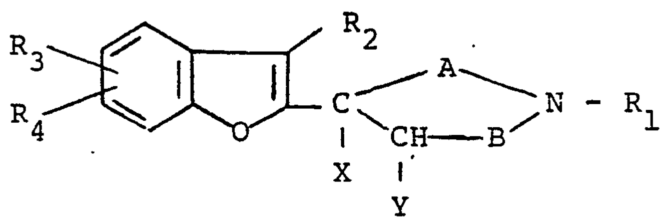

(II)

in welcher $R_1$ Wasserstoff, eine Alkylgruppe mit höchstens 4 Kohlenstoffatomen, die Allyl-, 3-Oxobutyl-, 3-Hydroxybutyl-, 2-Propinyl- oder Cyclopropylmethylgruppe, $R_2$ Wasserstoff oder eine Methylgruppe, $R_3$ Wasserstoff, Halogen bis Atomnummer 35, eine niedere Alkyl- oder Alkoxygruppe, die Trifluormethylgruppe oder eine Cycloalkylgruppe mit 5-8 Kohlenstoffatomen, $R_4$ Wasserstoff eine niedere Alkylgruppe oder Halogen bis Atomnummer 35, oder als $R_3$ und $R_4$ zusammen einen 1,3-Butadienylenrest in 4,5-Stellung, entsprechend einem ankondensierten Benzolring, oder einen Trimethylenrest in 5,6-Stellung, A die Aethylengruppe und B die Methylengruppe oder A die Methylengruppe und B die Aethylengruppe, und X und Y je Wasserstoff oder zusammen eine zusätzliche Bindung bedeuten, oder ein pharmazeutisch annehmbares Säureadditionssalz eines solchen enthalten.

2. Pharmazeutische Präparate nach Anspruch 1, dadurch gekennzeichnet, dass sie als in 4-Stellung basisch substituiertes Butyrophenonderivat I ein solches der allgemeinen Formel I

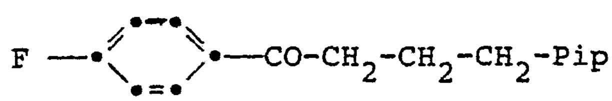

(I)

in welcher Pip einen 4,4-disubstituierten oder 4-substituierten Piperidinorest bedeutet, oder ein pharmazeutisch annehmbares Säureadditionssalz eines solchen enthalten.

3. Pharmazeutische Präparate nach Anspruch 2, dadurch gekennzeichnet, dass sie eine Verbindung der allgemeinen Formel I, in welcher Pip einen 4-Hydroxy-4-arylpiperidinorest, den 4-Carbamoyl-4-piperidino-piperidinorest oder den 4-(2-Oxo-2,3-dihydroxy-1H-benzimidazol-1-yl)-piperidinorest bedeutet, oder ein pharmazeutisch annehmbares Säureadditionssalz einer solchen Verbindung enthalten.

4. Pharmazeutische Präparate nach Anspruch 2, dadurch gekennzeichnet, dass sie eine Verbindung der allgemeinen Formel I, in welcher Pip einen in 4-Stellung durch Hydroxy und Halogenphenyl, Trifluormethylphenyl oder Niederalkylphenyl substituierten Piperidinorest bedeutet,

oder ein pharmazeutisch annehmbares Säureadditionssalz einer solchen Verbindung enthalten.

5. Pharmazeutische Präparate nach Anspruch 2, dadurch gekennzeichnet, dass sie eine Verbindung der allgemeinen Formel I, in welcher Pip den 4- Hydroxy-4-(p-chlorphenyl)-piperidino-, 4-Hydroxy-4-[m-(trifluormethyl)-phenyl]-piperidino- oder 4-Hydroxy-4-(p-toyl)-piperidino-rest, oder ein pharmazeutisch annehmbares Säureadditionssalz einer solchen Verbindung enthalten.

6. Pharmazeutische Präparate nach Anspruch 1, dadurch gekennzeichnet, dass sie als Verbindung der allgemeinen Formel II eine solche, in der $R_1$ Wasserstoff, ein Alkylrest mit höchstens 4 Kohlenstoffatomen, ein 2-Propinyl- oder Cyclopropylmethylrest, $R_2$ Wasserstoff, $R_3$ Wasserstoff, Halogen bis Atomnummer 35, eine Methyl-, Methoxy- oder Cyclohexylgruppe, $R_4$ Wasserstoff, eine Methylgruppe, Chlor oder Brom, A eine Aethylengruppe und B eine Methylengruppe ist und X und Y zusammen eine zusätzliche Bindung oder je ein Wasserstoffatom bedeuten, oder ein pharmazeutisch annehmbares Säureadditionssalz einer solchen Verbindung enthalten.

7. Pharmazeutische Präparate nach Anspruch 1, dadurch gekennzeichnet, dass sie als Verbindung der allgemeinen Formel II 4-(5,6-Dimethyl-2-benzofuranyl)-piperidin, oder 4-(6-Chlor-2-benzofuranyl)-piperidin oder ein pharmazeutisch annehmbares Säureadditionssalz einer solchen Verbindung enthalten.

8. Pharmazeutische Präparate nach Anspruch 1, dadurch gekennzeichnet, dass sie als Wirkstoffe ein zentraldämpfendes und antipsychotisches, in 4-Stellung basisch substituiertes Butyrophenonderivat I oder ein pharmazeutisch annehmbares Säureadditionssalz eines solchen, und eine Verbindung der im Anspruch 1 definierten allgemeinen Formel II oder ein pharmazeutisch annehmbares Säureadditionssalz einer solchen im Mengenverhältnis von 1 zu 2 bis 1 zu 100 enthalten.

9. Pharmazeutische Präparate nach Anspruch 1, dadurch gekennzeichnet, dass sie als Wirkstoffe ein zentraldämpfendes und antipsychotisches, in 4-Stellung basisch substituiertes Butyrophenonderivat I oder ein pharmazeutisch annehmbares Säureadditionssalz eines solchen, und eine Verbindung der im Anspruch 1 definierten allgemeinen Formel II oder ein pharmazeutisch annehmbares Säureadditionssalz einer solchen im Mengenverhältnis von 1 zu 4 bis 1 zu 50 enthalten.

10. Pharmazeutische Präparate nach Anspruch 1, dadurch gekennzeichnet, dass sie als Wirkstoffe Haloperidol oder ein pharmazeutisch annehmbares Säureadditionssalz desselben und 4-(5,6-Dimethyl-2-benzofuranyl)-piperidin oder ein pharmazeutisch annehmbares Säureadditionssalz desselben enthalten.

11. Pharmazeutische Präparate nach Anspruch 10, dadurch gekennzeichnet, dass sie die beiden Wirkstoffe im Mengenverhältnis von 1 zu 2 bis 1 zu 100 enthalten.

12. Pharmazeutische Präparate nach Anspruch 10, dadurch gekennzeichnet, dass sie die beiden Wirkstoffe im Mengenverhältnis von 1 zu 10 bis 1 zu 50 enthalten.

13. Pharmazeutische Präparate nach Anspruch 10, dadurch gekennzeichnet, dass sie in einer Doseneinheit oder in einer passenden Menge einer nicht-einzeldosierten Arzneiform den erstgenannten Wirkstoff in einer Menge von 0,25 bis 2,5 mg, und den zweitgenannten Wirkstoff in einer Menge von 5 bis 50 mg enthalten.

14. Pharmazeutische Präparate nach Anspruch 10, dadurch gekennzeichnet, dass sie in einer Doseneinheit oder in einer passenden Menge einer nicht-einzeldosierten Arzneiform den erstgenannten Wirkstoff in einer Menge von 0,5 bis 1,0 mg, und den zweitgenannten Wirkstoff in einer Menge von 10 bis 25 mg enthalten.

15. Pharmazeutische Präparate nach Ansprüchen 1 bis 14, gekennzeichnet durch einen zusätzlichen Gehalt an üblichen Träger- und/oder Hilfsstoffen.